# EUROPÄISCHE PATENTSCHRIFT

(11) **EP 0 810 902 B1**
(45) Veröffentlichungstag und Bekanntmachung des Hinweises auf die Patenterteilung: **21.10.1998**
(21) Anmeldenummer: 95924203.3
(22) Anmeldetag: 17.05.1995
(51) Int. Cl.: B01J 8/24, C07C 17/15, C07C 19/045

(54) **VORRICHTUNG UND IHRE VERWENDUNG ZUR OXICHLORIERUNG**
DEVICE AND USE THEREOF FOR OXI-CHLORINATION
DISPOSITIF ET SON UTILISATION POUR L'OXYCHLORATION

(30) Priorität: 20.02.1995 DE 19505664
(43) Veröffentlichungstag der Anmeldung: 10.12.1997
(73) Patentinhaber: HOECHST AKTIENGESELLSCHAFT, 65926 Frankfurt am Main (DE)
(72) Erfinder: KRUMBÖCK, Reinhard, D-84508 Burgkirchen (DE)
(74) Vertreter: Schuderer, Michael
(86) Internationale Anmeldenummer: EP9501874
(87) Internationale Veröffentlichungsnummer: WO9626003

(56) Entgegenhaltungen:
- EP-A- 0 320 904
- EP-A- 0 446 379
- WO-A-94/19099
- GB-A- 1 265 770

## Beschreibung

Unter "Oxichlorierung" wird die Umsetzung von Ethylen mit Chlorwasserstoff und Sauerstoff oder einem sauerstoffhaltigen Gas verstanden, wobei 1,2-Dichlorethan (EDC) entsteht. Als Chlorwasserstoff wird hierbei üblicherweise der bei der thermischen Spaltung von EDC zu Vinylchlorid anfallende Chlorwasserstoff genutzt.

Für die Oxichlorierung werden unter anderem Katalysatoren eingesetzt, die auf staubfeinen Trägern wie Aluminiumoxid Metallhalogenide, vorzugsweise Kupferchlorid, enthalten. Die Katalysatorteilchen haben hierbei einen mittleren Durchmesser von etwa 50 µm und bilden ein Fließbett aus, das entweder nur von den Reaktionsgasströmen, gegebenenfalls mit Inertgasanteilen, oder zusätzlich von einem Kreislaufgasstrom getragen wird. Bei diesem Verfahren wird die Reaktionswärme im Fließbett verteilt und an Kühlflächen abgeführt, wodurch eine gleichmäßige Temperaturverteilung im Fließbettreaktor erreicht wird. Hierbei müssen die Katalysatorteilchen eine hohe Abriebfestigkeit besitzen. Diese Eigenschaft wird im wesentlichen durch das Trägermaterial gegeben, wofür neben dem bereits genannten Aluminiumoxid auch Kieselsäure, Kieselgur oder Bimsstein eingesetzt werden. Bei ungenügender Abriebfestigkeit werden die Katalysatorteilchen zerrieben, insbesondere durch die Gasstrahlen der Gaseinleitungsvorrichtung, und der so auftretende Katalysatorträgerstaub wird vom aufwärtsgerichteten Gasstrom aus dem Oxichlorierungsreaktor ausgetragen. Hierdurch wird nicht nur ein Katalysatorverlust bewirkt, sondern auch eine erhöhte Abrasion in der Apparatur erzeugt.

Die Verwendung eines abriebfesten Trägermaterials bringt andererseits einen verstärkten Verschleiß der Gaseinleitvorrichtungen mit sich, der zu deren häufigem Auswechseln führt, was einen erheblichen Aufwand und zusätzliche Kosten durch die Produktionsunterbrechung bedeutet.

Neben der erforderlichen Abwägung zwischen der Stabilität der Katalysatorteilchen und der dadurch bedingten Abrasion muß auch noch darauf geachtet werden, daß die Katalysatorteilchen nicht agglomerieren, da hierdurch bedingte Verklumpungen zur Störung des Fließbettes führen. Die Folgen wären eine ungleichmäßige Temperaturverteilung im Fließbett mit entsprechend ungünstigerer Reaktionsführung sowie gegebenenfalls Verstopfungen an Engstellen in der Apparatur, beispielsweise in Zyklonen zur Staubabscheidung oberhalb des Fließbettes oder in Fallrohren zur Rückführung des Staubes aus diesen Zyklonen in das Fließbett. Diese Verklumpungsneigung hängt neben der Beschaffenheit des Katalysators und seiner Verteilung auf dem Katalysatorträger insbesondere von der Konzentration der Reaktionsgase im Fließbett ab.

Aus der EP-A-0 446 379 ist ein Reaktor zur Herstellung α-β-ungesättigter Nitrile bekannt, in dessen unterem Teil eine waagrecht angeordnete Gaszuführung für ein Olefin oder tertiären Butylalkohol vorgesehen ist, wobei an der Unterseite der Gaszuführung eine Vielzahl von Düsen angeordnet ist, sowie eine weitere Zuführung für ein Sauerstoff enthaltendes Gas, die unterhalb und parallel zu der anderen Gaszuführung angeordnet ist, wobei der Abstand zwischen den beiden Gaszuführungen 25 bis 300 mm beträgt. Ein zu kurzer Abstand kann zu einer Beschädigung der Gaszuführungen durch Schmelzen aufgrund einer ungewöhnlichen Reaktion führen, während bei zu langen Abständen das Olefin beziehungsweise der tertiäre Butylalkohol nicht ausreichend mit dem Sauerstoff enthaltenden Gas gemischt wird, was die Ausbeute an Nitril verringert.

Aus der GB-A-1 265 770 ist ein Reaktor für Fließbettreaktionen mit einer Verteilerplatte im unteren Teil des Reaktors bekannt, unter der eine Gaszuführung und im Randbereich des Reaktorgehäuses oberhalb und benachbart zu der Verteilerplatte eine weitere Gaszuführung vorgesehen ist. Diese weitere Gaszuführung bewirkt, daß sich im Randbereich der Katalysator nicht absetzt. Diese Vorkehrung dient insbesondere dazu, daß der Katalysator nicht reduziert wird. Innerhalb des Fließbetts kann eine weitere Gaszuführung vorgesehen sein, die im unteren Teil des Reaktors angeordnet ist, damit eine gute Durchmischung der Reaktanden an der Basis des Fließbetts erfolgt.

Aus WO 94/19099 ist eine Vorrichtung zur Oxichlorierung bekannt, die gekennzeichnet ist durch einen Reaktor 1, eine untere Begrenzung 2 für einen Fließbett-Katalysator 3, eine Gaseinleitung (Verteilerrohr) 4, die Düsen 5 enthält, wobei die Düsen 5 in Rohre 6 münden, die dem austretenden Gasstrom eine horizontale Komponente in der Strömungsrichtung verleihen, und eine Gaseinleitung 9 unterhalb der Begrenzung 2.

Bevorzugte Ausführungsformen richten sich darauf, daß die genannten Rohre, in die die Düsen münden, am Ende Lenkvorrichtungen mit Austrittsöffnungen enthalten, oder daß diese Rohre schräg nach oben oder in horizontaler Richtung oder schräg nach unten zeigen und diese Rohre frei im Katalysatorbett enden, oder daß diese Rohre beziehungsweise die Austrittsöffnungen benachbarter Rohre so angeordnet sind, daß die ausströmenden Gasstrahlen nicht frontal aufeinandertreffen und/oder nicht ein benachbartes Rohr treffen. Weitere bevorzugte Ausführungsformen richten sich darauf, daß durch die Begrenzung Rohre hindurchgeführt werden, in denen Düsen unterhalb der Begrenzung, aber oberhalb dem unteren Ende der durchgeführten Rohre angeordnet sind, wobei vorzugsweise diese Düsen unterhalb der halben Länge der betreffenden Rohre angebracht sind, insbesondere in der Entfernung von etwa einem Durchmesser des Rohres vom unteren Ende.

Es wurde nun gefunden, daß diese bekannte Vorrichtung bei langem Betrieb und hohen Durchsätzen, also hohen Gasgeschwindigkeiten, in den Gaseinleitungsvorrichtungen einen gewissen Abrieb aufweisen. Überraschenderweise wurde weiterhin gefunden, daß dieser Abrieb nicht oder nur in erheblich verringertem Umfang auftritt, wenn die Rohre, in welche die Düsen münden, den austretenden Gasstrom im wesentlichen entgegen dem Gasstrom führen, der den Katalysator in Form eines Fließbettes hält.

Die Erfindung betrifft somit eine Vorrichtung zur Oxichlorierung, gekennzeichnet durch
- einen Reaktor 1,
- eine untere Begrenzung 2 für ein Katalysator-Fließbett 3,
- oberhalb der Begrenzung 2 und innerhalb des Katalysator-Fließbettes 3 eine Gaseinleitung (Verteilerrohre) 4, die über den gesamten Querschnitt des Reaktors 1 verteilt Düsen 5 enthält,
- wobei die Düsen 5 in Rohre 6 münden, die den austretenden Gasstrom im wesentlichen im Gegenstrom zu dem Gasstrom führen, der den Katalysator fluidisiert und
- eine Gaseinleitung 7 unterhalb der Begrenzung 2.

Vorteilhaft wird die erfindungsgemäße Vorrichtung so ausgestaltet, daß der Anzahl von Rohren 8 die gleiche Anzahl von Rohren 6 gleichmäßig über den Querschnitt eines Reaktors 1 verteilt gegenüber angeordnet ist. Durch das Fluchten jeweils eines der Rohre 8 und 6 ist eine Zuordnung der Mengen miteinander reagierender Gase aus den Rohren 8 und 6 in besonderem Maße gegeben.

In einer anderen Ausführungsform der erfindungsgemäßen Vorrichtung liegen sich eine gleiche Anzahl von Rohren 8 und 6 versetzt gegenüber. Diese Geometrie bewirkt eine möglichst geringe Erosion der Rohre 6 durch den aus den Rohren 8 aufsteigenden Gasstrom. Weiters erreicht man mit dieser Anordnung, daß die aus den Rohren 8 und 6 in das Katalysator-Fließbett 3 eintretenden Reaktanden sofort einem innigen Kontakt mit dem Katalysator ausgesetzt sind. Dadurch wird die gewünschte Reaktion in Richtung Bildung von EDC begünstigt und Nebenreaktionen, wie zum Beispiel die Verbrennung des Ethylens mit dem Sauerstoff, zurückgedrängt.

Eine weitere Ausführungsform dieser Vorrichtung läßt für ihre Auslegung und Konstruktion größere Freiräume zu. Hierbei ist die Anzahl der Rohre 6 von der Anzahl der Rohre 8 verschieden. Wichtig ist aber auch hier die möglichst gleichmäßige Verteilung dieser Rohre über den Querschnitt des Reaktors 1. Diese Form ergibt die Möglichkeit, die Anzahl der Rohre 6 in einem vorhandenen Reaktor zu verändern, ohne zugleich die Rohre 8 anzupassen, was einen hohen Aufwand bedeuten würde.

Die Erfindung wird in dem folgenden Beispiel noch näher erläutert.

### Beispiel

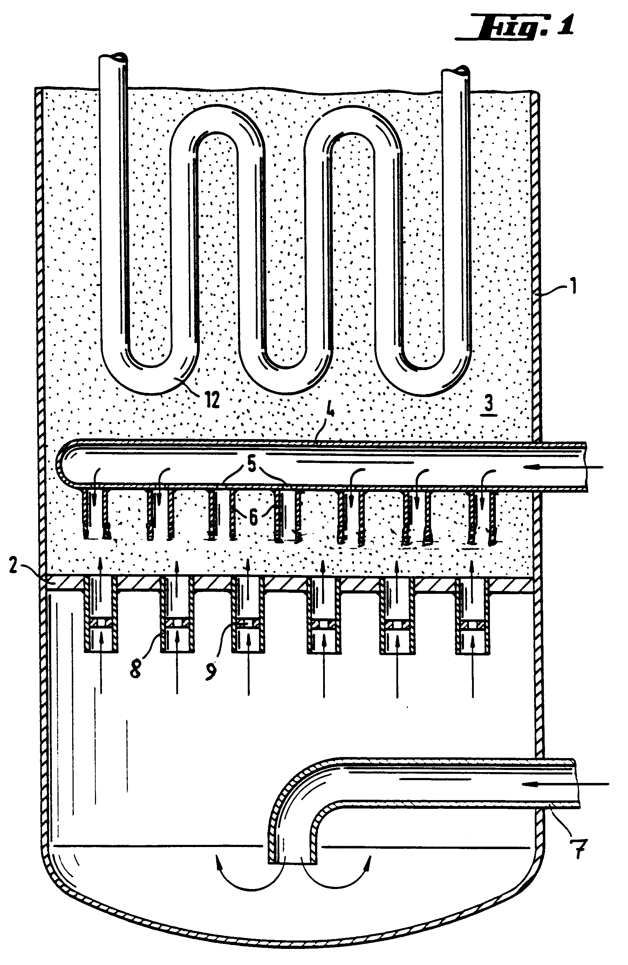

Es wird eine Apparatur gemäß Figur 1 verwendet. In einen Reaktor 1 von 2,8 m Durchmesser und 26 m Höhe werden die auf 160 °C vorgewärmten Reaktanden gasförmig eingeleitet. Eine Mischung aus 5974 kg/h Chlorwasserstoff und 1417 kg/h Sauerstoff strömt durch die Gaseinleitung 4 über die Düsen 5 und die Rohre 6 in das Katalysator-Fließbett 3. Die Düsen 5 besitzen verschiedene Durchmesser, damit eine möglichst gleichmäßige Gasverteilung auf alle Düsen 5 und somit über den Querschnitt des Reaktors 1 erreicht wird. Der Durchmesser der Düsen 5 vergrößert sich stromabwärts der Gaseinleitung 4 von 8,6 mm auf 9,3 mm und weiter auf 10 mm, um die verschiedenen Druckverluste entlang der Gaseinleitung bis zu den jeweiligen Düsen 5 zu kompensieren. Die Rohre 6 mit einem Innendurchmesser von 40 mm haben eine Länge von 300 mm. 2380 kg/h Ethylen fließen über die Gaseinleitung 7 und die Rohre 8 mit den Düsen 9 durch die untere Begrenzung 2. Im Reaktor 1 befindet sich als Katalysator Kupfer-(II)-chlorid auf einem Aluminiumoxid-Träger in der Form eines Katalysator-Fließbettes 3. In dieses Fließbett werden die obengenannten Reaktanden eingeführt. Zur Fluidisierung des Fließbettes strömt zusätzlich ein Kreisgasstrom von 8780 kg/h über die Gaseinleitung 7 und die Rohre 8 durch die untere Begrenzung 2 in den Reaktor 1. Die oberen Enden der Rohre 8 sind bündig mit der unteren Begrenzung 2. Der Abstand zwischen dieser unteren Begrenzung und den unteren Enden der Rohre 6 beträgt 400 mm. In diesem Abschnitt werden die Reaktanden über den Reaktorquerschnitt verteilt, und es bilden sich Mischungszonen zwischen den einzelnen Reaktanden und dem Katalysator. Ethylen und Kreisgas strömen im Reaktor von unten nach oben. Auf diesem Weg treffen sie auf den Chlorwasserstoff und Sauerstoff und reagieren unter Mitwirkung des vorhandenen Katalysators zu EDC und Wasser. Die dabei auftretende Reaktionswärme von 238,5 kJ/mol wird über das Katalysator-Fließbett 3 an die Kühlschlange 12 abgeführt, in der Wasser bei 183 °C verdampft. Die Reaktionstemperatur beträgt 225 °C bei einem Überdruck von 3,2 bar im Reaktor. Der Gasstrom am Reaktorkopf, bestehend aus den Reaktionsprodukten und dem Kreisgas, verläßt den Reaktor 1 über drei Zyklone zur weiteren Verarbeitung (nicht in der Figur dargestellt). Die drei in Reihe geschalteten Zyklone dienen zur Abscheidung des mitgerissenen Katalysatorstaubes aus dem Gasstrom am Reaktorkopf oberhalb des Katalysator-Fließbettes.

## Patentansprüche

1. Vorrichtung zur Oxichlorierung, gekennzeichnet durch
- einen Reaktor (1),
- eine untere Begrenzung (2) für ein Katalysator-Fließbett (3),
- oberhalb der Begrenzung (2) und innerhalb des Katalysator-Fließbettes (3) eine Gaseinleitung (Verteilerrohre) (4), die über den gesamten Querschnitt des Reaktors (1) verteilt Düsen (5) enthält,
- wobei die Düsen (5) in Rohre (6) münden, die den austretenden Gasstrom im wesentlichen im Gegenstrom zu dem Gasstrom führen, der den Katalysator fluidisiert,
- eine Gaseinleitung (7) unterhalb der Begrenzung (2) und
- durch die Begrenzung (2) hindurchgeführte Rohre (8),
- wobei der Raum zwischen den oberen Enden der durch die Begrenzung (2) hindurchgeführten Rohre (8) und den unteren Enden der Rohre (6), in die die Düsen (5) münden, eine Mischzone bildet, die so groß bemessen ist, daß die Vermischung der aus diesen Rohren austretenden Reaktanden mit dem Katalysator hier bereits stattfinden kann, wobei eine Mischzone von 25 bis 300 mm ausgeschlossen ist.

2. Vorrichtung nach Anspruch 1, gekennzeichnet durch durch die Begrenzung (2) hindurchgeführte Rohre (8), in denen Düsen (9) unterhalb der Begrenzung (2), aber oberhalb dem unteren Ende von (8) angeordnet sind.

3. Vorrichtung nach Anspruch 2, dadurch gekennzeichnet, daß die Düsen (9) in der Entfernung von etwa einem Durchmesser der Rohre (8) vom unteren Ende der Rohre (8) angebracht sind.

4. Vorrichtungen nach Anspruch 1, dadurch gekennzeichnet, daß die Düsen (5) unterschiedliche Durchmesser haben, damit die über die Gaseinleitung (4) geführte Gasmenge gleichmäßig über den Querschnitt des Reaktors (1) verteilt wird.

5. Verwendung der Vorrichtungen nach den Ansprüchen 1 bis 4 für die Umsetzung von Ethylen mit Chlorwasserstoff und Sauerstoff oder einem sauerstoffhaltigen Gas zu 1,2-Dichlorethan.

## Claims

1. Apparatus for oxychlorination, characterized by
- a reactor (1),
- a lower delimitation (2) for a catalyst fluid bed (3),
- a gas inlet (distributor tubes) (4) above the delimitation (2) and within the catalyst fluid bed (3), which gas inlet contains nozzles (5) distributed over the entire cross-section of the reactor (1),
- the nozzles (5) opening into tubes (6) which conduct the exiting gas stream essentially in countercurrent to the gas stream which fluidizes the catalyst,
- a gas inlet (7) beneath the delimitation (2) and
- tubes (8) run through the delimitation (2),
- the space between the upper ends of the tubes (8) run through the delimitation (2) and the lower ends of the tubes (6), into which the nozzles (5) open, forming a mixing zone which is dimensioned so that the reactants exiting from these tubes can already mix with the catalyst here, a mixing zone of 25 to 300 mm being excluded.

2. Apparatus according to Claim 1, characterized by tubes (8) run through the delimitation (2), in which tubes nozzles (9) are arranged beneath the delimitation (2), but above the lower end of (8).

3. Apparatus according to Claim 2, characterized in that the nozzles (9) are installed at a distance of about one diameter of the tubes (8) from the lower end of the tubes (8).

4. Apparatuses according to Claim 1, characterized in that the nozzles (5) have different diameters, so that the gas conducted via the gas inlet (4) is uniformly distributed over the cross-section of the reactor (1).

5. Use of the apparatuses according to Claims 1 to 4 for the reaction of ethylene with hydrogen chloride and oxygen or an oxygen-containing gas to give 1,2-dichloroethane.

## Revendications

1. Dispositif d'oxychloration, caractérisé en ce qu'il comprend :
- un réacteur (1);
- une délimitation inférieure (2) pour un lit fluidisé de catalyseur (3);
- au-dessus de la délimitation (2) et à l'intérieur du lit fluidisé de catalyseur (3), une arrivée de gaz (tuyaux répartiteurs) (4), qui comprend des gicleurs (5) répartis sur tout le profil du réacteur (1);
- les gicleurs (5) débouchant dans les tuyaux (6), qui conduisent le courant gazeux sortant essentiellement à contre-courant du courant gazeux, qui fluidifie le catalyseur;
- une arrivée de gaz (7) au-dessous de la délimitation (2) et
- des tuyaux (8) passés au travers de la délimitation (2),
- l'espace entre les extrémités supérieures des tuyaux (8) passés au travers de la délimitation (2) et les extrémités inférieures des tuyaux (6), dans lesquels les gicleurs (5) débouchent, formant une zone de mélange qui est d'une taille telle que le mélange des réactifs sortant de ces tuyaux et du catalyseur peut déjà y avoir lieu, une zone de mélange de 25 à 300 mm étant isolée.

2. Dispositif suivant la revendication 1, caractérisé en ce que les tuyaux (8) passés au travers de la délimitation (2), dans lesquels des gicleurs (9) sont disposés au-dessous de la délimitation (2) mais au-dessus de l'extrémité inférieure de (8).

3. Dispositif suivant la revendication 2, caractérisé en ce que les gicleurs (9) sont placés à une distance de l'extrémité inférieure des tuyaux (8) d'à peu près un diamètre des tuyaux (8).

4. Dispositifs suivant la revendication 1, caractérisés en ce que les gicleurs (5) ont des diamètres différents afin que la quantité gazeuse amenée par l'arrivée de gaz (4) soit répartie de manière homogène sur le profil du réacteur (1).

5. Utilisation des dispositifs suivant les revendications 1 à 4, pour la réaction de l'éthylène avec le chlorure d'hydrogène et l'oxygène, ou un gaz contenant de l'oxygène, pour donner le 1,2-dichloroéthane.
